# EUROPEAN PATENT APPLICATION

(11) **EP 3 785 540 A1**
(43) Date of publication of application: **03.03.2021**
(21) Application number: 20193545.9
(22) Date of filing: 31.08.2020
(51) Int. Cl.: A01N 27/00, A01P 7/00

(54) **POLYISOBUTENE FOR USE IN TREATMENT OR PREVENTION OF INFECTION BY ARTHROPODS**

(30) Priority: 30.08.2019 BE 201905574
(71) Applicant: Oystershell NV, 9820 Merelbeke (BE)
(72) Inventor: Rossel, Bart, 9820 Merelbeke (BE)
(74) Representative: Brantsandpatents bvba

(57) **Abstract**

The present invention provides polyisobutene and composition thereof and their use medical, insecticidal, prophylactic or therapeutic substances for the treatment or prevention of infection or infestation by arthropods. More specifically, the invention relates to the use of polyisobutene and compositions thereof for treatment of lice infections.

## Description

### FlELD OF THE INVENTION

The present invention relates to the field of polyisobutene compounds, composition thereof and their use as medical, insecticidal or therapeutic substances. More specifically, the invention relates to the use of polyisobutene and compositions thereof for treatment of arthropod infection.

### BACKGROUND

Many compounds with adulticidal or ovicidal activity in arthropods are known. However, many of these compounds have downsides such as being difficult to obtain, difficult to purify, difficult to apply, difficult to wash out, not being suitable for use in humans in particular children, and so forth.

EP 2 334 186 relates to the non-therapeutic use of a composition containing vitamin E or an ester thereof for the control of ectoparasites, in particular lice and nits. Said composition comprises a solvent selected from the group consisting of volatile siloxanes, hydrogenated polyisobutene, hydrogenated polydecene and mixtures of hydrogenated polyisobutene and/or hydrogenated polydecene with hydrogenated polyolefines. EP 2 334 186 does not disclose adulticidal or ovicidal activity of polyisobutene on arthropods. EP 2 334 186 related to the addition of specialty chemicals such as vitamin E as active ingredients.

EP 2 101 756 discloses an avermectin compound for the manufacture of a medicament for topical application on an affected skin area for treating hyperesthesia, paresthesia, or dolor caused by a sting by bees, wasps, hornets, yellow jackets, or fire ants. Polyisobutene is described as a component of a moisturizing agent, to be used as the carrier of said avermectin compound. EP 2 101 756 does not disclose adulticidal or ovicidal activity of polyisobutene on arthropods.

US 8 945 516 is related to a substantially surface-active agent free composition which includes a hydrophobic solvent, and/or a petrolatum, a paraffin wax and/or a fatty alcohol, a fatty acid and/or a wax and/or shea butter, with and without a propellant. Polyisobutene is disclosed in a list of suitable hydrophobic solvents, hydrocarbon oils or waxes. In an embodiment, said composition in combination with an active ingredient is used in the treatment of lice.

The invention thereto aims to provide a novel compound and composition thereof for the treatment and prevention of arthropod infection.

### SUMMARY OF THE I NVENTI ON

The present invention and embodiments thereof serve to provide a solution to one or more of above-mentioned disadvantages. To this end, the present invention relates to polyisobutene for the use in treatment or prevention of arthropod infection in humans or animals according to claim 1.

Preferred embodiments of the device are shown in any of the claims 2 to 6. A specific preferred embodiment relates to an invention according to claim 6. The invention is particularly suited for the treatment or prevention of lice.

In an aspect, the present invention relates to a composition according to claim 9. More particular, the use as described herein provides a composition for use in the treatment of arthropods comprising polyisobutene as active ingredient. Polyisobutene, particularly compared to many active ingredients, is well tolerated. Polyisobutene has low occurrence of allergies and negative reactions. Accordingly, the composition comprising a hydrophobic carrier can be applied directly to an area to be treated, preventively or curatively. Polyisobutene has surprisingly found to be an effective preventive treatment. Furthermore, it scored high on consumer acceptance as a hair product particularly. Consumers rated it highly for washing out effectively, granting a good hair gloss as well as obtaining non-greasy hair.

Preferred embodiments of the composition are shown in any of the claims 8 to 14.

In another aspect the present invention relates to a method according to claim 15. The method as described herein can advantageously be used to prepare a composition according to any of claims 7-14.

### DETAILED DESCRIPTION OF THE INVENTION

The present invention concerns polyisobutene for use in treatment or prevention of infection by arthropods in humans or animals.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

As used herein, the following terms have the following meanings:
"A", "an", and "the" as used herein refers to both singular and plural referents unless the context clearly dictates otherwise. By way of example, "a compartment" refers to one or more than one compartment.
"About" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1% or less of and from the specified value, in so far such variations are appropriate to perform in the disclosed invention. However, it is to be understood that the value to which the modifier "about" refers is itself also specifically disclosed.
"Comprise", "comprising", and "comprises" and "comprised of" as used herein are synonymous with "include", "including", "includes" or "contain", "containing", "contains" and are inclusive or open-ended terms that specifies the presence of what follows e.g. component and do not exclude or preclude the presence of additional, non-recited components, features, element, members, steps, known in the art or disclosed therein.
   Furthermore, the terms first, second, third and the like in the description and in the claims, are used for distinguishing between similar elements and not necessarily for describing a sequential or chronological order, unless specified. It is to be understood that the terms so used are interchangeable under appropriate circumstances and that the embodiments of the invention described herein are capable of operation in other sequences than described or illustrated herein.
   The recitation of numerical ranges by endpoints includes all numbers and fractions subsumed within that range, as well as the recited endpoints.
The expression "% by weight", "weight percent", "%wt" or "wt%", here and throughout the description unless otherwise defined, refers to the relative weight of the respective component based on the overall weight of the formulation.
   An acrylate (co)polymer is an acrylate polymer and / or an acrylate copolymer.
Whereas the terms "one or more" or "at least one", such as one or more or at least one member(s) of a group of members, is clear *per se,* by means of further exemplification, the term encompasses *inter alia* a reference to any one of said members, or to any two or more of said members, such as, e.g., any ≥3, ≥4, ≥5, ≥6 or ≥7 etc. of said members, and up to all said members.

Unless otherwise defined, all terms used in disclosing the invention, including technical and scientific terms, have the meaning as commonly understood by one of ordinary skill in the art to which this invention belongs. By means of further guidance, definitions for the terms used in the description are included to better appreciate the teaching of the present invention. The terms or definitions used herein are provided solely to aid in the understanding of the invention.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to a person skilled in the art from this disclosure, in one or more embodiments. Furthermore, while some embodiments described herein include some, but not other features included in other embodiments, combinations of features of different embodiments are meant to be within the scope of the invention, and form different embodiments, as would be understood by those in the art. For example, in the following claims, any of the claimed embodiments can be used in any combination.

### 1. Polyisobutene

In a first aspect, the invention provides polyisobutene, or a pharmaceutically acceptable substituted form thereof.

The inventors have surprisingly found that such compounds exhibit a high adulticidal and ovicidal activity in arthropods. In particular, such compounds exhibit a high pediculicidal activity, thereby effectively exterminating lice. This is of special interest since such compounds are easily obtainable compared to active ingredients commonly used for pediculicidal activity i.e. silicone-based agents. Furthermore, polyisobutene is well tolerated by humans and animals compared to active ingredients commonly used for pediculicidal activity i.e. lindane, ivermectin, malathion, phenothrin, permethrin, dimethicone. Without being bound to any mechanistic theories, it is rationalized that polyisobutene and other apolar substances affect lice and other arthropods because these substances penetrate and cover the breathing organs of these animals (such as the spiracles, trachea, gills or primitive lungs) and interfere with respiration and gas/moisture exchange. In addition, these substances stick to the exoskeleton and may have other arthropod impairing effects. The activity of these substances hinges on their non-solubility: when covered with these substances, the animals are unable to solubilize or expel them from their organs and skeleton. An advantage of such adulticidal and ovicidal activity is that the arthropods are unlikely or unable to develop resistance or immunity for such products, unlike insecticides such as permethrin, DDT and malathion. However, the drawback of these effective apolar substances is that this insolubility is inconvenient when used on the treated person's hair or treated animal's hair or feathers. Surprisingly, polyisobutene in particular in a composition according to the invention was found to be effective against arthropods such as head lice while they were easily washed out after the topical treatment. This is a significant advantage over compounds known in the art, such as silicone-based agents as dimethicone. Moreover, compositions comprising polyisobutene showed a conditioning effect to the treated hairs, thereby providing an easy and complete hair treatment to the consumer. By not requiring further active ingredients for the treatment or prevention of arthropods, negative side effects of these active ingredients such as allergies, negative impact on hair or skin, chemical interactions with other substances, expensive to produce and short shelf-life can be avoided.

In a preferred embodiment, the present invention provides polyisobutene according to the first aspect of the invention, having a molecular weight lower than 10 000 g/mol, preferably lower than 8 000 g/mol, more preferably lower than 6 000 g/mol, more preferably lower than 5 000 g/mol, more preferably lower than 4 000 g/mol, more preferably lower than 3 000 g/mol, more preferably lower than 2 000 g/mol, i.e. between 200 g/mol and 2 000 g/mol. Preferably, said polyisobutene has a molecular weight between 500 g/mol and 1 500 g/mol, and more preferably between 700 g/mol and 1300 g/mol. Even more preferably, said polyisobutene has a molecular weight higher than 100 g/mol, preferably higher than 200 g/mol, more preferably higher than 300 g/mol, more preferably higher than 400 g/mol, more preferably higher than 500 g/mol, more preferably higher than 600 g/mol, more preferably higher than 700 g/mol, more preferably higher than 800 g/mol, more preferably higher than 900 g/mol. Most preferably, said polyisobutene has a molecular weight of about 925 g/mol, 950 g/mol, 975 g/mol, 1 000 g/mol, 1 025 g/mol, 1 050 g/mol, 1 075 g/mol or 1 100 g/mol. The inventors have found that a sufficiently low molecular weight allows for polyisobutene to be washed out after application more easily. Without being bound by any mechanistic theories, a sufficiently high molecular weight is required to obtain desirable adulticidal and ovicidal activity.

Polyisobutene according to the present invention is a polymer prepared by polymerization of isobutene. In an embodiment, polyisobutene according to the present invention is prepared from and comprises over 90% by weight of isobutene monomer, preferably over 92% by weight of isobutene monomer, more preferably over 94% by weight of isobutene monomer, more preferably over 95% by weight of isobutene monomer, more preferably over 96% by weight of isobutene monomer, more preferably over 97% by weight of isobutene monomer, more preferably over 98% by weight of isobutene monomer, more preferably over 99% by weight of isobutene monomer, more preferably over 99.1% by weight of isobutene monomer, more preferably over 99.2% by weight of isobutene monomer, more preferably over 99.3% by weight of isobutene monomer, more preferably over 99.4% by weight of isobutene monomer, more preferably over 99.5% by weight of isobutene monomer, more preferably over 99.6% by weight of isobutene monomer, more preferably over 99.7% by weight of isobutene monomer, more preferably over 99.8% by weight of isobutene monomer, more preferably over 99.9% by weight of isobutene monomer.

The "pharmaceutically acceptable copolymers and derivatives" as mentioned hereinbefore or hereinafter are meant to comprise the therapeutically active non-toxic copolymers of isobutene comprising at least 90% by weight of isobutene monomer, as well as any non-toxic derivatives such as halogenated forms or salts thereof. The pharmaceutically acceptable copolymers by copolymerizing isobutylene with at least a second monomer, oligomer or polymer. Said copolymer can subsequently be treated, for example hydrogenated, halogenated or functionalized.

Pharmaceutically acceptable salts can be obtained by producing a base form, for example by functionalizing with amines or alcohols, and subsequently treating said base form of the functionalized polyisobutene with appropriate acids, for example inorganic acids, for example hydrohalic acid, in particular hydrochloric acid, hydrobromic acid, sulfuric acid, nitric acid and phosphoric acid; organic acids, for example acetic acid, hydroxyacetic acid, propanoic acid, lactic acid, pyruvic acid, oxalic acid, malonic acid, succinic acid, maleic acid, fumaric acid, malic acid, tartaric acid, citric acid, methanesulfonic acid, ethanesulfonic acid, benzenesulfonic acid, p-toluenesulfonic acid, cyclamic acid, salicylic acid, p-aminosalicylic acid and pamoic acid. The "pharmaceutically acceptable copolymers and derivatives" according to the invention containing acidic protons may be converted into their therapeutically active non-toxic metal or amine addition salt forms by treatment with appropriate organic and inorganic bases. The term "pharmaceutically acceptable salt" also comprises the quaternary ammonium salts (quaternary amines) which the polyisobutene copolymer according to the invention are able to form by reaction between a basic nitrogen in the main chain of the polyisobutene copolymer and an appropriate quaternizing agent, such as, for example, an optionally substituted C1-6 alkyl halide, aryl C1-6 alkyl halide, C1-6 alkyl carbonyl halide, aryl carbonyl halide, Het-C1-6 alkyl halide or Het-carbonyl halide, e.g. methyl iodide or benzyl iodide. Preferably, "Het" represents a monocyclic heterocycle selected from furanyl or thienyl; or a bicyclic heterocycle selected from benzofuranyl or benzothienyl; each monocyclic and bicyclic heterocycle may optionally be substituted with 1, 2 or 3 substituents, each substituent independently selected from the group of halogen, alkyl and aryl. 5 Preferably, the quaternizing agent is C1-6 alkyl halide. Other reactants with good leaving groups may also be used, such as C1-6 alkyl trifluoromethane sulfonates, C1-6 alkyl methane sulfonates, and C1-6 alkyl p-toluene sulfonates. A quaternary amine has a positively charged nitrogen. Pharmaceutically acceptable counter ions include chloro, bromo, iodo, trifluoro acetate, acetate, triflate, sulfate, sulfonate. Preferably, the counter ion is iodo. The counter ion of choice can be introduced using ion exchange resins.

The term "solvate" comprises the hydrates and solvent addition forms which polyisobutene according to the invention is able to form, as well as the salts thereof. Examples of such forms are e.g. hydrates, alcoholates and the like.

The safety of polyisobutene and hydrogenated polyisobutene has been shown for cosmetics. According to the "Final report of the cosmetic ingredient review expert panel on the safety assessment of Polyisobutene and Hydrogenated Polyisobutene as used in cosmetics" (International Journal of toxicology 2008;27 Suppl 4:83-106. doi: 10.1080/10915810802550611).

Polybutene is a chemically related cosmetic ingredient previously determined to be safe as used in cosmetic products. Polyisobutene is used in cosmetic products as a binder, film former, and nonaqueous viscosity-increasing agent. Hydrogenated Polyisobutene functions as a skin-conditioning agent-emollient and nonaqueous viscosity-increasing agent with a wide range of uses in cosmetic formulations.

Acute oral toxicity testing demonstrated no effects other than lethargy in one rat study. The oral LD(50) was > 5.0 g/kg in rats. No short-term or subchronic animal toxicity data were available.

Neither Polyisobutene nor Hydrogenated Polyisobutene were ocular irritants, nor were they dermal irritants or sensitizers. Polyisobutene was not comedogenic in a rabbit ear study. In a carcinogenicity study in mice, Polyisobutene was not carcinogenic, nor did it promote the carcinogenicity of 7,12-dimethylbenz(alpha)anthracene. Clinical patch tests uncovered no evidence of dermal irritation and repeat-insult patch tests with a product containing 4% Hydrogenated Polyisobutene or 1.44% Hydrogenated Polyisobutene found no reactions greater than slight erythema. These products also were not phototoxic or photo-allergenic. The product containing 4% Hydrogenated Polyisobutene was not classified as an ocular irritant.

Because lifetime exposure studies using rats and dogs exposed to polybutene failed to demonstrate any carcinogenic or tumor promotion effect, and a three-generation reproductive/developmental toxicity study produced no adverse effects, the CIR Expert Panel does not believe these large, mostly insoluble polymers present any risks in the practices of use and concentration as described in this safety assessment.

In the framework of this application, a polyisobutene according to the invention is inherently intended to comprise all isotopic combinations of its chemical elements. In the framework of this application, a chemical element, in particular when mentioned in relation to a polyisobutene according to the invention, comprises all isotopes and isotopic mixtures of this element, either naturally occurring or synthetically produced, either with natural abundance or in an isotopically enriched form. In particular, when hydrogen is mentioned, it is understood to refer to ¹H, ²H, ³H and mixtures thereof; when carbon is mentioned, it is understood to refer to ¹¹C, ¹²C, ¹³C, ¹⁴C and mixtures thereof; when nitrogen is mentioned, it is understood to refer to ¹³N, ¹⁴N, ¹⁵N and mixtures thereof; when oxygen is mentioned, it is understood to refer to ¹⁴O, ¹⁵O, ¹⁶O, ¹⁷O, ¹⁸O and mixtures thereof; and when fluoro is mentioned, it is understood to refer to ¹⁸F, ¹⁹F and mixtures thereof. A compound according to the invention therefore inherently comprises a compound with one or more isotopes of one or more element, and mixtures thereof, including a radioactive compound, also called radio labelled compound, wherein one or more nonradioactive atoms has been replaced by one of its radioactive isotopes. By the term "radio labelled compound" is meant any polyisobutene according to the invention, a pharmaceutically acceptable salt thereof or an N-oxide form thereof or a solvate thereof, which contains at least one radioactive atom. For example, a compound can be labelled with positron or with gamma emitting radioactive isotopes. For radio ligand binding techniques (membrane receptor assay), the ³H-atom or the ¹²⁵I-atom is the atom of choice to be replaced. For imaging, the most commonly used positron emitting (PET) radioactive isotopes are ¹¹C, ¹⁸F, ¹⁵O and ¹³N, all of which are accelerator produced and have half-lives of 20, 100, 2 and 10 minutes respectively. Since the half-lives of these radioactive isotopes are so short, it is only feasible to use them at institutions which have an accelerator on site for their production, thus limiting their use. The most widely used of these are ¹⁸F, ²⁰¹TI and ¹²³I. The handling of these radioactive isotopes, their production, isolation and incorporation in a molecule are known to the skilled person. In particular, the radioactive atom is selected from the group of hydrogen, carbon, nitrogen, sulfur, oxygen and halogen. Preferably, the radioactive atom is selected from the group of hydrogen, carbon and halogen. In particular, the radioactive isotope is selected from the group of ³H, ¹¹C, ¹⁸F, ¹²²I, ¹²³I, ¹²⁵I, ¹³¹I, ⁷⁵Br, ⁷⁶Br, ⁷⁷Br and ⁸²Br. Preferably, the radioactive isotope is selected from the group of ³H, ¹¹C and ¹⁸F.

### 2. Use of polyisobutene

In a second aspect, the present invention provides polyisobutene according to the first aspect of the invention for use as a treatment solution.

In a third aspect, the present invention provides polyisobutene for use in treatment or prevention of infection or infestation by arthropods. The term "arthropod" refers to members of the arthropod phylum, including, but not limited to the hexapod insects but also ticks, spiders, scorpions, centipedes, millipedes. Insects include, but are not limited to the species in the orders of diptera, phthiraptera, heteroptera, hemiptera, hymenoptera, blattodea, trichoptera, lepidoptera, orthoptera, thriptera and coleopteran.

In accordance with the present invention, polyisobutene as defined herein are used for killing arthropods and their eggs. The term "killing" as used herein includes repelling, reducing in number, and eradicating said arthropods, e.g. ectoparasites, and/or their eggs. Use of the present polyisobutene for killing arthropods and/or their eggs includes prophylactic use.

Polyisobutene or compositions thereof according to the present invention are useful in the killing of arthropods, particularly terrestrial arthropods, especially insects and arachnids, and their eggs. Insects include ectoparasites. Ectoparasites include sucking and biting lice, fleas, keds, mites and ticks. In particular, said polyisobutene or compositions thereof have pediculicidal activity, and are therefore especially useful for treating infestations of lice in animals, including humans. More preferably, said polyisobutene or compositions thereof can advantageously be used for the treatment or prevention of infection by lice. Most preferably, polyisobutene or compositions thereof can be used for the treatment or prevention of head lice in humans.

Sucking lice (Anoplura) and biting lice (Mallophaga, a paraphyletic name for Rhyncophthirina, Ischnocera and Amblycera) are parasites found on nearly all groups of mammals and birds. They include such species as *Haematopinus* spp., *Linognathus* spp., *Solenopotes* spp., *Pediculus* spp., and *Pthirus* spp. *Pediculus* spp. include *Pediculus humanus,* e. g. the head louse *Pediculus humanus capitis* and the body or clothing louse *Pediculus humanus corporis.* Pthirus spp. includes the crab louse *Pthirus pubis.*

In a preferred embodiment, the invention relates to polyisobutene for use in treatment or prevention of infection or infestation by arthropods in humans or animals. In a more preferred embodiment, the invention relates to polyisobutene for use in treatment or prevention of infection or infestation by lice in humans or animals. In a more preferred embodiment, the invention relates to polyisobutene for use in treatment or prevention of infection or infestation by lice in humans. In a more preferred embodiment, the invention relates to polyisobutene for use in treatment or prevention of infection or infestation by head lice in humans.

Effective adulticidal and ovicidal are well known in the state of the art. However, certain issues remain. Head lice have been known to become immune to certain remedies. For example, according to "Resistance and cross-resistance to insecticides in human head lice from Florida and California" published in Pesticide Biochemistry and Physiology, Volume 80, November 2008, head lice from Florida (SF-HL) and California (SC-HL) were resistant to permethrin compared to lice from Panama and Ecuador. These lice were cross resistant to pyrethrum and DDT. For these resistant or immune lice, alternative remedies are required. It is believed arthropods do not build resistance to polyisobutene due to its physical mode of action.

A second issue is that infections or infestations of arthropods such as ticks and lice are frequently found in children. Many pediculicidal agents and insecticides are not suited for medical use on children. Some agents can be used, but preferably not frequently or often. A pediculicidal agent which is safe to use on children is desirable. Furthermore, child-safe agents are desired. In particular this relates to agents which are not toxic when ingested in small doses, which do not hurt or cause severe irritation upon contact with eyes or skin. Polyisobutene is considered safe for use.

A third issue is that infections or infestations of arthropods, in particular lice are often spread through gatherings or groups of humans or animals (e.g. schools, youth movements, ...). While various agents that effectively kill lice and nits are known in the art, few agents that prevent infection or infestation from the environment are known. This leads to an infection or infestation often persisting for several weeks until the infection or infestation of arthropods has been removed from (almost) all humans or animals in the school or gathering. The inventors surprisingly found that polyisobutene repelled oviposition and generally prevented infestation or infection by arthropods. This is very desirable to avoid the spreading, infection or potential re-infection or infestation of arthropods in groups of humans or animals. Advantageously, polyisobutene can be applied to humans and animals, in particular children, sequentially throughout one, two, three, four or more weeks if needed or desirable. This is beneficial to prevent and combat the spreading of lice as well as preventing infection or infestation by lice in children.

Ticks are the largest group of the subclass Acari and are obligate blood-sucking ectoparasites of land vertebrates. Certain species are pests of domestic livestock, while another group transmits human disease. Ticks are classified into three families, all but one species belonging to the *Ixodidae* (hard ticks) for to the *Argasidae* (soft ticks). The present polyisobutene and compositions thereof can be used to kill soft as well as hard ticks.

Polyisobutene or compositions thereof are also useful for the control of other arthropods, including for example public health pests e.g. cockroaches and bed bugs; nuisance arthropods e. g. wasps, ants, silver fish and woodlice; and structural pests e. g. furniture beetles, deathwatch beetles and other wood borers.

Arthropod eggs include eggs of ectoparasites as defined herein and include but are not limited to eggs of sucking and biting lice -also called nits or ova-, eggs of fleas, keds, mites and ticks.

Preferably, polyisobutene or compositions thereof are used on mammalian skin and avian feathers, respectively. On humans, it is applicable to the treatment of hair and scalp. Human hair care products according to the present invention can be used in conventional ways and generally involve the application of an effective amount of the hair product onto wet or dry hair, preferably on dry hair, and on the scalp. Polyisobutene or compositions thereof are left in/on the hair for about 5 minutes to 15 minutes and are subsequently removed by rinsing and effortlessly washing the hair. Surprisingly, polyisobutene or compositions thereof were found to be effective against arthropods such as head lice despite being easily washed out after the topical treatment. This is a significant advantage over compounds and compositions known in the art, such as dimethicone. Products comprising dimethicone are known to be left in/on the hair for up to 8 hours before. Moreover, compositions comprising said polyisobutene showed a conditioning effect to the treated hairs, thereby providing an easy and complete hair treatment to the consumer. In particular hydrogenated polyisobutene shows a conditioning effect to treated hair and skin. Polyisobutene or compositions thereof are distributed throughout the hair, typically by rubbing or massaging the hair and scalp with ones' hands or by another's hands. An effective amount of the composition comprising 1 to 10% by weight polyisobutene, typically from about 1 gram to about 100 grams, preferably from about 10 grams to about 30 grams, is applied.

Polyisobutene or compositions thereof of the present invention may be used in a conventional manner.

An effective amount of the composition comprising 1 to 10% by weight polyisobutene, typically from about 1 gram to about 200 grams, for instance from about 30 grams to about 150 grams or from about 1 gram to about 100 grams, or from about 10 grams to about 30 grams is applied.
The method for treating hair for instance comprises the steps of: (a) applying an effective amount of polyisobutene or compositions thereof to the wet or dry hair and the scalp, (b) working polyisobutene or compositions thereof in contact with the hair and scalp, especially to the region behind the ears and the hairline at the back of the head, (c) leaving polyisobutene or compositions thereof on the hair for a suitable period of time to allow killing to occur and (d) rinsing polyisobutene or compositions thereof from the hair using water alone or water and soap. Application of polyisobutene or compositions thereof to the hair typically includes working polyisobutene or compositions thereof through the hair, generally with the hands and fingers. Polyisobutene or compositions thereof is left into contact with the hair, e.g. for about 5 or 10 minutes to 15 minutes. Polyisobutene or compositions thereof is then rinsed from the hair with water and optionally soap (e.g. a shampoo).

The method for treating the skin for instance comprises the steps of: (a) applying an effective amount of polyisobutene or compositions thereof to the skin, (b) leaving polyisobutene or compositions thereof on the skin for a suitable period of time to allow killing to occur and (c) rinsing polyisobutene or compositions thereof from the skin using water or water and soap. Polyisobutene or compositions thereof can be left into contact with the skin, e.g. for about 5 or 10 minutes to 15 minutes. Polyisobutene or compositions thereof is then rinsed from the skin with water and optionally soap.

The method steps can be repeated as many times as desired to achieve the sought effects. But preferably the treatment is repeated after 7 days. To kill off the hatched lice that were not killed in the first treatment.

### 3. Compositions comprising polyisobutene

In a fourth aspect, the present invention provides a composition comprising polyisobutene according to the first aspect of the invention and a suitable carrier.

In a preferred embodiment, the composition comprises polyisobutene as sole arthropodically active ingredient. The inventors have found that polyisobutene as sole arthropodically active ingredient provides sufficient activity in the treatment of arthropod infestation. There is no need to add further arthropodicides. In fact adding further arthropodicides, biocides, insecticides or pediculicides is generally not desired. These substances often lead to undesired effects such as irritation of skin or hair, developing resistance of arthropods infestations to said arthopodicide and reduced shelf-life of the composition. In particular, the composition preferably does not comprise : organophosphates and organochlorides such as lindane and malathion; carbamates and carboxamides such as carbaryl; organosilicones such as dimethicone and silylated polyalcohols, pyrethrins and pyrethroids such as pyrethrum, piperonyl butoxide, permethrin, phenothrin, bioallethrin, resmethrin, cyhalothrin, deltamethrin; avermectins and deratives thereof such as ivermectin, selamectin, doramectin, eprinomectin, moxidectin, and abamectin; milbemycins and deratives thereof, vitamin E and esters thereof; moxidectin, butopyronoxyl, 2,3;4,5-bis(2- butylene)tetra- hydro-2- furaldehyde, N,N- diethylcapryl- amide, o-chloro-N,N-di- ethylbenzamide in a mixture with N,N-diethyl- benzamide, dimethyl carbate, di-n-propyl iso- cinchomeronate, N-octylbicyclo- heptenedicarbox- imide, piperonyl butoxide, 2-ethylhexane- 1,3-diol and spinosad.

In a preferred embodiment, the present invention provides a composition according to the fourth aspect of the invention, wherein said polyisobutene is comprised in an amount of 0.1 to 25 wt.%. Preferably, said polyisobutene is comprised in an amount of 0.5 to 15 wt.% and more preferably in an amount of 1 to 10 wt.%. Most preferably, said polyisobutene is comprised in an amount of 2, 3, 4, 5, 6 or 7 wt.%.

In a preferred embodiment, the present invention provides a composition according to the fourth aspect of the invention, wherein said polyisobutene is a mixture of two or more polyisobutenes. Preferably, said polyisobutene is a mixture of a partially or completely hydrogenated polyisobutene.

### Carrier

In a preferred embodiment, the present invention provides a composition according to the fourth aspect of the invention, wherein said carrier is hydrophobic, as determined by an AlogP98 value higher than 0.000 and preferably higher than 1.000. In a preferred embodiment, the present invention provides a composition according to the fourth aspect of the invention, wherein said carrier has an AlogP98 value higher than 1.000 and lower than 10.000, and more preferably higher than 1.000 and lower than 6.000. Even more preferably, said carrier has an AlogP98 value of about 1.000, 1.500, 2.000, 2.500, 3.000, 3.500 or 4.000 or any value there in between. Examples of suitable carriers are non-toxic hydrophobic solvents. Examples of suitable hydrophobic carriers are selected from the group comprising of a diglyceride, a PPG alkyl ether, a therapeutic oil, acetylated lanolin alcohol, alexandria laurel tree oil, alkyl benzoate, alkyl octanoate, almond oil, an essential oil, an unsaturated or polyunsaturated oil, apricot stone oil, arachidyl behenate, arachidyl propionate, avocado oil, barley oil, basil oil, beeswax, benzyl laurate, benzyl myristate, benzyl palmitate, bis(octyldodecyl stearoyl) dimer dilinoleate, borage seed oil, butyl myristate, butyl stearate, C12-C15 alkyl benzoate, C12-C15 alkyl octanoate, calendula oil, camphor oil, canelle nut tree oil, canola oil, capric/caprylic triglycerides, caprylic/capric triglyceride castor oil, caprylyl methicone, cardamom oil, carrot oil, castor oil, cetearyl ethylhexanoate, cetearyl isononanoate, cetearyl octanoate, cetyl acetate, cetyl dimethicone, cetyl ethylhexanoate, cetyl lactate, cetyl myristate, cetyl octanoate, cetyl palmitate, cetyl ricinoleate, citronella oil, clary sage oil, clove oil, cocoglycerides, coconut oil, cod-liver oil, corn oil, cotton oil, cottonseed oil, cyclohexasiloxane, cyclomethicone, Cyclomethicone 5-NF (cyclopentasiloxane), cyclotetrasiloxane, cypress oil, decyl oleate, diethyleneglycol diethylhexanoate, diethyleneglycol diisononanoate, diethyleneglycol dioctanoate, diethylhexanoate, diethylhexyl adipate, diethylhexyl malate, diethylhexyl succinate, diisopropyl adipate, diisopropyl dimerate, diisopropyl sebacate, diisosteary dimer dilinoleate, diisostearyl fumerate, dimethicone, dimethyl polysiloxane, dioctyl malate, dioctyl sebacate, disopropyl adipate, dodecyl oleate, cyclotetrasiloxanecyclohexasiloxane and cyclopentasiloxane mixtures, epoxy-modified silicone oil, essential oils, ester derivatives of lanolic acid, ester oils, ethylhexyl cocoate, ethylhexyl ethylhexanoate, ethylhexyl hydroxystarate, ethylhexyl isononanoate, ethylhexyl palmitate, ethylhexyl palmytate, ethylhexyl pelargonate, ethylhexyl stearate, evening primrose oil, fatty acid-modified silicone oil, flaxseed oil, fluoro group-modified silicone oil, frankincense oil, gelled mineral oil, ginger oil, glycereth triacetate, glycerol triheptanoate, glyceryl oleate, glyceryl trioctanoate, glyceryl triundecanoate, grape seed oil, grapefruit oil, groundnut oil, hard fat, hazelnut oil, heavy mineral oil, hempseed oil, herring oil, hexadecyl stearate, hexyl laurate, hydrocarbon oils, hydrogenated castor oil, hyssop oil, isoamyl laurate, isocetearyl octanoate, isocetyl isocetyl behenate, isocetyl lanolate, isocetyl palmitate, isocetyl salicylate, isocetyl stearate, isocetyl stearoyl stearate, isodecyl ethylhexanoate, isodecyl isononanoate, isodecyl oleate, isododecane, isohexadecane isododecane, isohexadecanol, isohexyl decanoate, isononyl isononanoate, isononyl octanoate, isoparaffin or isoparaffinic oil, isopropyl isostearate, isopropyl lanolate, isopropyl laurate, isopropyl myristate, isopropyl palmitate, isopropyl stearate, isosteary citrate, isosteary salicylate, isosteary tartarate, isostearyl behenate, isostearyl erucate, isostearyl glycolate, isostearyl isononanoate, isostearyl isostearate, isostearyl lactate, isostearyl linoleate, isostearyl linolenate, isostearyl malate, isostearyl neopentanoate, isostearyl palmitate, isotridecyl isononanoate, jasmine oil, jojoba oil, lauryl lactate, lavender oil, lemon oil, light mineral oil, liquid paraffin, liquid triglycerides, lucerne oil, maize germ oil, maleated soybean oil, mandarin oil, manuka oil, marjoram oil, marrow oil, MCT oil, methylphenylpolysiloxane, millet oil, mineral oil, myristyl lactate, myristyl myristate, myristyl neopentanoate, myristyl propionate, myrrh oil, neopentylglycol dicaprate, neopentylglycol dicaprylate/dicaprate, neroli oil, nutmeg oil, octyl palmitate, octyl stearate, octyldodecanol, octyldodecyl behenate, octyldodecyl hydroxystearate, octyldodecyl myristate, octyldodecyl stearoyl stearate, oils from animal origin, oils of plant origin, oleyl erucate, oleyl lactate, oleyl oleate, olive oil, or dimethiconol, palm oil, passionflower oil, peanut oil, PEG/PPG 18/18 dimethicone, pentaerythrityl tetrastearate, petitgrain oil, petrolatum, phenyl trimethicone, phenyltrimethicone, poly(dimethylsiloxane)-(diphenyl-siloxane) copolymer, polyalkyl siloxane, polyalkylaryl siloxane, polyalphaolefin, polyaryl siloxane, polyaryl siloxanes, polyether group-modified silicone oil cyclomethicone, polyether siloxane copolymer, polyether siloxane copolymers, polyisobutylene, polyolefin, poppy oil, PPG alkyl ethers, PPG-10 cetyl ether, PPG-10 oleyl ether, PPG-11 stearyl ether, PPG-12 butyl ether, PPG-14 butyl ether, PPG-15 butyl ether, PPG-15 stearyl ether, PPG-16 butyl ether, PPG-17 butyl ether, PPG-18 butyl ether, PPG-2 butyl ether, PPG-2 methyl ether, PPG-20 butyl ether, PPG-20 oleyl ether, PPG-22 butyl ether, PPG-23 oleyl ether, PPG-24 butyl ether, PPG-26 butyl ether, PPG-28 cetyl ether, PPG-3 methyl ether, PPG-3 myristyl ether, PPG-30 butyl ether, PPG-30 cetyl ether, PPG-30 isocetyl ether, PPG-30 oleyl ether, PPG-33 butyl ether, PPG-37 oleyl ether, PPG-4 butyl ether, PPG-4 lauryl ether, PPG-4 myristyl ether, PPG-40 butyl ether, PPG-5 butyl ether, PPG-50 cetyl ether, PPG-50 oleyl ether, PPG-52 butyl ether, PPG-53 butyl ether, PPG-7 lauryl ether, PPG-9 butyl ether, PPG-9-13 butyl ether, propyl myristate, propylene glycol dicaprate, propylene glycol dicaprylate, propylene glycol myristyl ether acetate, propylene glycol ricinoleate, rapeseed oil, rosehip oil, rye oil, safflower oil, sage oil, salmon oil, sesame oil, shea butter, silicone oils, soya oil, soybean oil, stearyl caprate, stearyl dimethicone, stearyl heptanoate, stearyl propionate, sunflower oil, sweet almond oil, synthetic isoalkane, sysymbrium oil, syzigium aromaticum oil, tangerine oil, tea tree oil, therapeutic oils, tocopheryl acetate, tocopheryl linoleate, tridecyl ethylhexanoate, tridecyl isononanoate, triisocetyl citrate, unsaturated or polyunsaturated oils, vanilla oil, verbena oil, walnut oil, wheat germ glycerides, wheat germ oil, white petrolatum and mixtures thereof.

In a preferred embodiment, the present invention provides a composition according to the fourth aspect of the invention, wherein said carrier is an oil, such as a mineral oil, a vegetable oil, an essential oil, or any combination of two or more of the aforementioned oils. Mineral oils are known to the person skilled in the art as alkanes from a mineral source and mixtures thereof. Typical examples of vegetable oils are almond oil, apricot kernel oil, avocado oil, borage seed oil, camellia seed oil (tea oil), coconut oil (fractionated), coconut oil (virgin), cranberry seed oil, evening primrose oil, grapeseed oil, hazelnut oil, hemp seed oil, jojoba, kukui nut oil, macadamia nut oil, meadowfoam oil, olive oil, peanut oil, pecan oil, pomegranate seed oil, rose hip oil, seabuckthorn berry oil, sesame oil, sunflower oil and watermelon seed oil. Essential oils are known to the person skilled in the art as concentrates of aromatic compounds obtained from plants. In a more preferred embodiment, the carrier is isoparaffinic oil. Isoparaffinic oil has desirable properties to carry polyisobutylene in a desired amount and sufficiently low viscosity. Isoparaffinic oil is non-toxic and suitable for use in medicaments. Isoparaffinic oil has a long shelf-life compared to many essential oils and vegetable oils. Furthermore, isoparaffinic oil is colorless and odourless, which is desirable over the odour of many organic solvents.

In a preferred embodiment, the carrier comprises C6-C24 hydrocarbons, preferably C8-C22 hydrocarbons, more preferably C8-C22 hydrocarbons, more preferably C10-C20 hydrocarbons, more preferably C12-C20 hydrocarbons, more preferably C14-C20 hydrocarbons, most preferably C14-C19 hydrocarbons. Said hydrocarbons are preferably isoparaffins or cyclic. Said hydrocarbon mixture preferably comprises less than 5.0 wt.% aromatics, more preferably less than 3.0 wt.% aromatics, more preferably less than 2.0 wt.% aromatics, more preferably less than 1.5 wt.% aromatics, more preferably less than 1.0 wt.% aromatics, more preferably less than 0.5 wt.% aromatics, more preferably less than 0.1 wt.% aromatics, more preferably less than 0.01 wt.% aromatics, more preferably roughly no aromatics, most preferably no aromatics. Many aromatics are not suitable for use in medicinal compositions and are often irritating to the skin.

In a preferred embodiment, the present invention provides a composition according to the fourth aspect of the invention, having a viscosity lower than 25.0 Pa.s at 25°C and preferably lower than 20.0 Pa.s at 25°C. Preferably, said carrier has a viscosity lower than 15.0 Pa.s, 12.0 Pa.s, 10.0 Pa.s, 8.0 Pa.s or 5.0 Pa.s at 25°C. Most preferably, said carrier has a viscosity of about 0.8 Pa.s, 1.0 Pa.s, 1.2 Pa.s, 1.4 Pa.s, 1.6 Pa.s, 1.8 Pa.s, 2.0 Pa.s, 2.2 Pa.s, 2.4 Pa.s or 2.6 Pa.s at 25°C, or any value there in between. Without being bound by any mechanistic theories, the inventors assume that a sufficiently low viscosity allows for a good distribution of the polyisobutene in the composition to the subject. Hence, a good contact with the to be treated arthropods is achieved and a high efficiency of treatment is obtained. In accordance, experiments indicate that carriers with lower viscosity exhibit higher ovicidal and adulticidal activity.

### Additives

In addition to the essential components described hereinbefore, the present compositions may further comprise one or more optional components that are known or otherwise suitable for use on human/animal hair or skin. Non-limiting examples of such optional components include for instance plasticizers and humectants, free radical scavengers, viscosity-adjusting agents, dyes and colorants, perfumes, preservatives and the like.

In a preferred embodiment, the composition is a lotion according to the fourth aspect of the invention is a lotion. A lotion is a low-viscosity topical preparation intended for application to the skin or hair on humans or animals. In a further preferred embodiment, the composition according to the fourth aspect of the invention does not comprise a foaming agent in an amount high enough to produce foam. In a further preferred embodiment, the lotion is absent of foaming agents in an amount sufficient to produce a foam and wherein said lotion is absent of surfactants in an amount sufficient to produce an emulsion. In a further preferred embodiment, the composition does not comprise any foaming agents chosen from the list of : sodium coceth sulfate, sodium lauryl sulfate, sodium bicarbonate, sodium lauryl ether sulfate, ammonium coceth sulfate, ammonium lauryl sulfate, ammonium bicarbonate, ammonium lauryl ether sulfate, oleaginous foamer complexes, liquefied gas propellants, compressed gas propellants. In a further preferred embodiment, the composition does not comprise a foaming agent. In a preferred embodiment, polyisobutene dissolves or gels in the hydrophobic carrier producing a lotion with sufficiently low viscosity. In another preferred embodiment, the composition is not an emulsion of polyisobutene and an immiscible carrier. In a further preferred embodiment, the composition does not comprise surfactant or emulsifier in an amount high enough to produce a polyisobutene emulsion. The inventors have found that foams and emulsions of polyisobutene reduce the arthropodicidal activity.

In a particularly preferred embodiment, in addition to the essential components described herein may further comprise one or more optional components that are suitable for the prevention of arthropod infection. Without being bound to theory, certain terpenes and other perfumes comprised in essential oils creates an odor unattractive to lice. Accordingly, these terpenes and perfumes repel lice. In a more preferred embodiment, the composition comprises an essential oil. In a more preferred embodiment, the composition comprises an essential oil of flowers.

Similarly, certain film-forming substances coat hair fibers in such a way that the surface structure becomes unattractive to lice. Accordingly, these film-forming substances repel lice. In a more preferred embodiment, acrylate polymer, acrylate copolymer and / or refined sesame oil can be added as film-forming repellent. These repellents are added to the composition in an amount of 0.1 to 5% by weight, preferably in an amount of 0.2 to 3% by weight, most preferably in an amount of 0.3, 0.4, 0.5, 0.6, 0.7, 0.8, 0.9, 1.0, 1.1, 1.2, 1.3, 1.4, 1.5, 1.6, 1.7, 1.8, 1.9 % by weight of the composition.

In a preferred embodiment, the compositions according to the invention are stored at room temperature, and preferably at a temperature below room temperature, such as at a temperature below 20°C, below 15°C, below 10°C or below 5°C. Preferably, said compositions are stored at a temperature of about 4°C. For reasons of effective handling, it is preferred that the compositions are stored at a temperature above the melting temperature of the carrier.

In a particularly preferred embodiment, the composition is a lotion comprising polyisobutene in C14-C19 isoparaffinic oil. In a more preferred embodiment, the composition comprises polyisobutene in an amount of 2.0 to 6.0 wt.% of polyisobutene in C14-C19 isoparaffinic oil. In a more preferred embodiment, the comprises polyisobutene and acrylate copolymer in C14-C19 isoparaffinic oil. Preferably said acrylate copolymer is comprised in an amount of 0.2 to 1 wt.% relative to the weight of the composition. In another preferred embodiment, the composition comprises polyisobutene and sesame oil in C14-C19 isoparaffinic oil. Preferably said sesame oil is comprised in an amount of 1.0 to 2.0 wt.% relative to the weight of the composition. In a further preferred embodiment, the composition comprises :
- polyisobutene, in an amount of 1.0 to 8.0 wt.% relative to the weight of the composition,
- acrylate (co)polymer in an amount of 0.1 to 2.0 wt.% relative to the weight of the composition,
- refined sesame oil, in an amount of 0.5 to 4.0 wt.% relative to the weight of the composition,
- optionally one or more essential oil in an combined amount of 0.05 to 2.0 wt.% relative to the weight of the composition, and
- supplemented to 100 wt.% with C14-C19 isoparaffinic oil.
In a more preferred embodiment, the composition comprises :
- polyisobutene, in an amount of 2.0 to 4.0 wt.% relative to the weight of the composition,
- acrylate (co)polymer in an amount of 0.2 to 1.0 wt.% relative to the weight of the composition,
- refined sesame oil, in an amount of 1.0 to 2.0 wt.% relative to the weight of the composition,
- optionally one or more essential oil in an combined amount of 0.05 to 2.0 wt.% relative to the weight of the composition, and
- supplemented to 100 wt.% with C14-C19 isoparaffinic oil.
These composition was found to be sufficiently active to kill arthropods and eggs thereof. Furthermore, this composition was found to have a repelling effect for arthropods. The composition was also well-tolerated and liked with regards to customer satisfaction, hair look, volume and gloss and could be rinsed out easily.

In a preferred embodiment, the composition consists of polyisobutene, a film-forming polymer, optionally an oil, optionally (an) essential oil(s), and a lipophilic carrier. More preferably, the composition consists of polyisobutene, a film-forming polymer, sesame oil, optionally one or more essential oils, and a lipophilic carrier. More preferably, the composition consists of polyisobutene, a film-forming polymer, refined sesame oil, optionally one or more essential oils, and C14-C19 isoparaffinic oil. More preferably, the composition consists of polyisobutene, an acrylate (co)polymer, refined sesame oil, optionally one or more essential oils, and a lipophilic carrier. More preferably, the composition consists of polyisobutene, an acrylate (co)polymer, refined sesame oil, optionally one or more essential oils, and C14-C19 isoparaffinic oil. More preferably, the composition consists of polyisobutene, an acrylate (co)polymer, refined sesame oil, essential oil of flowers, and C14-C19 isoparaffinic oil. More preferably the composition consists of :
- polyisobutene, in an amount of 1.0 to 8.0 wt.% relative to the weight of the composition,
- acrylate (co)polymer in an amount of 0.1 to 2.0 wt.% relative to the weight of the composition,
- refined sesame oil, in an amount of 0.5 to 4.0 wt.% relative to the weight of the composition,
- optionally one or more essential oil in an combined amount of 0.05 to 2.0 wt.% relative to the weight of the composition, and
- supplemented to 100 wt.% with C14-C19 isoparaffinic oil.
In the most preferred embodiment, the composition is a lotion consisting of :
- polyisobutene, in an amount of 2.0 to 4.0 wt.% relative to the weight of the composition,
- acrylate (co)polymer in an amount of 0.2 to 1.0 wt.% relative to the weight of the composition,
- refined sesame oil, in an amount of 1.0 to 2.0 wt.% relative to the weight of the composition,
- optionally one or more essential oil in an combined amount of 0.05 to 2.0 wt.% relative to the weight of the composition, and
- supplemented to 100 wt.% with C14-C19 isoparaffinic oil.

### 4. Use of compositions comprising polyisobutene

In a fifth aspect, the present invention provides a composition according to the fourth aspect of the invention for use as a medicament.
In a sixth aspect, the present invention provides a composition according to the fourth aspect of the invention for use in treatment or prevention of infection or infestation by arthropods. The term "arthropod" refers to members of the arthropod phylum, including the hexapod insects but also other arthropods from diverse subphyla such as ticks, spiders, mites, wood lice, centipedes, millipedes. Insects include, but are not limited to the life stages of hematophagous or nuisance species from but not limited to the orders of diptera, phthiraptera, heteroptera, hemiptera, hymenoptera, blattodea, trichoptera, lepidoptera, orthoptera, thriptera and coleopteran. In accordance with the present invention, the compositions as defined herein are used for killing arthropods and/or their eggs, nymphs and juvenile life stages. The term "killing" as used herein includes repelling, reducing in number, and eradicating said arthropods, e.g. ectoparasites, and/or their eggs. Use of the present compositions for killing arthropods and/or their eggs includes prophylactic use.

In a preferred embodiment, the present invention provides a composition according to the fourth aspect of the invention for use in treatment or prevention of infection or infestation by lice in humans or animals. More preferably, the present invention provides a composition according to the fourth aspect of the invention for use in treatment or prevention of infection or infestation by lice in humans. Most preferably, the present invention provides a composition according to the fourth aspect of the invention for use in treatment or prevention of infection or infestation by head lice in humans.

### 5. Preparation of polyisobutene and compositions thereof

In a seventh aspect, the present invention provides a method for preparing a composition according to the fourth aspect of the invention comprising the step of mixing one or more polyisobutene polymers according to the first aspect of the invention with a suitable carrier to form a composition according to the fourth aspect of the invention.

The invention is further described by the following non-limiting examples which further illustrate the invention, and are not intended to, nor should they be interpreted to, limit the scope of the invention.

### EXAMPLES 1-7

Human head lice (Pediculus humanus capitis) were originally collected from infested children. These human head lice are believed to be permethrin-, DDT- and malathion resistant.
The tested compositions are shown in Table 1. Herein, examples 1 and 2 are examples according to the present invention. Examples 3-6 are comparative examples. Example 7 is a negative control. The C14-C19 hydrocarbons which are used as carrier are hydrotreated hydrocarbons with less than 2% aromatics.

**Table 1 : Composition of examples 1 - 7**

| EXAMPLE | Composition |
|---|---|
| 1 | 4.0 wt.% of polyisobutene in C14-C19 hydrocarbons |
| 2 | 4.0 wt.% of polyisobutene, 0.5 wt.% of acrylate copolymer and 1.5 wt.% refined sesame oil in C14-C19 hydrocarbons |
| 3 | 4.0 wt.% of dimethicone in C14-C19 hydrocarbons |
| 4 | 4.0 wt.% of isopropyl myristate in C14-C19 hydrocarbons |
| 5 | 1.0 wt.% of permethrin in C14-C19 hydrocarbons |
| 6 | 0.5 wt.% of malathion in C14-C19 hydrocarbons |
| 7 | Tap water |

### Pediculicidal activity

For the test procedure an aliquot of approximately lice were counted into batches and provided with squares of closed meshed nylon gauze, as a substrate upon which to stand. Each batch was allocated to a marked Petri dish.
An aliquot of 5-10 milliliters of each formulation was poured into the base of a clean Petri dish. The gauze bearing the lice was immersed into the fluid for 10 seconds, during which time the gauze was turned at least twice to ensure the removal of air bubbles. After removal from the fluid the gauze and insects were lightly bluffed to remove excess fluid and returned to a clean marked petri dish.
Gauze squares bearing the lice were then incubated under normal maintenance conditions, being 30°C and 50% relative humidity for the remainder of the test period. At the end of exposure period the insects and gauze were washed using a bland frequent use toiletry shampoo diluted one part shampoo with fourteen parts water after which they were rinsed using 500 milliliters of 35°C tap water poured through and over the gauze squares. They were then blotted dry using medicinal wipe tissue and incubated under normal maintenance conditions.

**Table 2 : Pediculicidal activity of examples 1 - 7**

| EXAMPLE | % Mortality one hour after washing off | % Mortality two hours after washing off |
|---|---|---|
| 1 | 100 | 100 |
| 2 | 100 | 100 |
| 3 | 100 | 100 |
| 4 | 98 | 100 |
| 5 | 62 | 67 |
| 6 | 38 | 41 |
| 7 | 14 | 18 |

Results shown in Table 2 demonstrate that examples 1-4 obtained close to 100% mortality within one hour after being washed off. The considerably lower immortality rates of examples 5 and 6 confirm that these head lice are resistant to permethrin and malathion.

Results show that the present invention matches has sufficient pediculicidal activity. In fact, each of the commercial state of the art products, barring those for which the lice have developed resistance, have a high pediculicidal activity.

### Oviposition repellency

A semicircular shape of flattened hair tuft (3 cm diameter) was placed on a glass Petri dish and treated with the composition of example 2 (1 g / g hair tuft) by slow and gentle application onto the hair tuft using a pipette gun. Using sterile forceps, the hair tuft was gently rubbed to complete hair tuft coverage with example 2 for 30 seconds and visually inspected to insure saturation under a stereomicroscope. Treated hair tuft was transferred to a clean petri dish and incubated for 15 minutes at room temperature. Following incubation, the treated hair tuft was sequentially washed using three separate 12 wt.% sodium lauryl sulfate baths for 30 seconds per wash and sequentially rinsed using three separate water baths for 30 seconds per rinse.

A control hair tuft treated with tap water (example 7, negative control) for 15 minutes at room temperature, and was washed and rinsed in the same way as described above. For effective treatment and wash, temperature of the 12 wt.% sodium lauryl sulfate solution and water was maintained at 25-30°C. The hair tufts were then air-dried on a stack of filter paper for 1 hour.

Only freshly prepared hair tufts were used in repellency test. A repellency test arena was constructed on the membrane surface of a rearing unit on the in vitro rearing system. Adult female lice (at least 3 days old, 10 lice/test) were placed on the boundary between polyisobutene-treated (example 2) and control (example 7) treated hair tufts. Number of female lice and eggs on each semi-circular hair tuft was recorded to determine 24, 48 and 72 hr repellency. Each of the two experiments (treatment and control) was replicated three times, respectively.

After repellency bioassays, individual hair tufts with eggs were placed into covered sterile glass Petri dishes and moved to an incubator at 31°C and 70-80% relative humidity. The number of lice that hatched from eggs were recorded and used to determine the percent hatchability of eggs. Undeveloped eggs and stillborn lice were recorded as dead.

There were significantly higher ovipositional repellency responses (93.6-94.1%) of adult female lice on the hair tufts treated with example 2. This high ovipositional repellency was consistent over a 72 hr period (P > 0.05). Interestingly, a skewed ovipositional behavior was observed from the control group (Table 3). This skewed behavior was also consistent over a 72 hr period (P > 0.05).

None of the eggs on the polyisobutene-treated hair tufts developed into 1st instars, and only small number (1.5-13.3%) of eggs on the control-treated hair tufts developed into 1st instars (Table 4).

**Table 3 : Oviposition repellency of examples 2 and 7**

| Time (hours) | Hair tuft treated with example 2, mean % +- SD | Hair tuft treated with example 7, mean % +- SD |
|---|---|---|
| 24 hr | 94.1 ± 6.13 | 64.2 ± 2.2 |
| 48 hr | 93.6 ± 3.33 | 62.2 ± 5.7 |
| 72 hr | 93.8 ± 4.33 | 66.5 ± 0.3 |

**Table 4 : Oviposition and egg hatchability of examples 2 and 7**

| Replication | Treatment group | | Control group | |
|---|---|---|---|---|
| | Example 2 | Example 7 | Example 7 | Example 7 |
| 1 | 0% (0/7) | 3.2% (2/63) | 5.3% (1/19) | 10.7% (6/56) |
| 2 | 0% (0/3) | 3.9% (4/103) | 11.3% (11/97) | 13.3% (4/30) |
| 3 | 0% (0/3) | 1.5% (1/65) | 12.5% (2/16) | 8.3% (4/48) |

### Consumer acceptance

Example 2 according to the invention and example 3 according to the state of the art were tested for consumer acceptance.

The study provided two samples in neutral, transparent bottles with applicator. Each product receives a random 3-digit reference code. Two samples were provided to 61 Belgian (Flemish) correspondents chosen from a database of 3000 volunteers. The samples are provided with clear instructions. The first lotion is tested at time 0. The second lotion is tested at time +2 weeks. The participants can test the lotions on their own hair or on the hair of their children, however both lotions were to be used on the same person.

Half the group first received example 2 and used example 3 two weeks later, the other half first received example 3 and then used example 2 two weeks later.

After using the lice treatment lotions, the correspondents wash their hair with their usual shampoo. They then were asked to evaluate their dried hair. Evaluations were measured on a 9-point system as well as relative-to-ideal scores.

**Table 5 : Acceptance score for examples 2 and 3**

| Acceptance score (out of 9, higher scores are "better") | Example 2 | | | Example 3 | | |
|---|---|---|---|---|---|---|
| | Average | St. Dev. | TOP-4 | Average | St. Dev. | TOP-4 |
| General feel of the hair | 6,16 | 1,74 | 72% | 6,05 | 1,90 | 69% |
| Feel of the scalp | 6,38 | 1,24 | 70% | 6,16 | 1,29 | 72% |
| Look of the hair | 6,08 | 1,92 | 70% | 5,95 | 1,94 | 69% |

**Table 6 : Relative-to-ideal scores for examples 2 and 3**

| Relative-to-ideal scores (%) | Example 2 | | | Example 3 | | |
|---|---|---|---|---|---|---|
| | Too much | Optimum | Too little | Too much | Optimum | Too little |
| Greasiness | 52 | 48 | | 71 | 30 | |
| Gloss | 28 | 56 | 16 | 31 | 51 | 18 |
| Volume | 2 | 56 | 43 | 3 | 56 | 41 |

The results are shown in Table 5 and
Table 6. Both examples 2 and 3 score well in terms of a good feel of hair and scalp and the general look of the hair. There are no statistically significant differences between both groups.

In terms of greasiness, hair volume and hair gloss statistically significant differences were measured between both lotions. Furthermore, 40% of the correspondents noted that the lotion of example 3 does not wash out easily. For example 2, only 21% of the correspondents had issues with washing out the lotion.

The consumer acceptance of example 2 was significantly better than that of example 3. Particularly the greasiness, hair volume and hair gloss and the ability of rinse off the lotion were better received for example 2.

## Claims

1. Polyisobutene for use in treatment or prevention of infection by arthropods in humans or animals.

2. Polyisobutene according to claim 1, wherein said polyisobutene has a molecular weight comprised between 100 and 10 000 g/mol.

3. Polyisobutene according to any of claims 1-2, wherein said polyisobutene has a molecular weight comprised between 200 and 5 000 g/mol.

4. Polyisobutene according to any of claims 1-3, wherein said polyisobutene is hydrogenated.

5. Polyisobutene according to any of claims 1-4, for use in treatment or prevention of infection by arthropod eggs.

6. Polyisobutene according to any of claims 1-5, for use in treatment or prevention of infection of lice.

7. Composition comprising polyisobutene according to claim 1-6 and a hydrophobic carrier for use in treatment or prevention of infection by arthropods in humans or animals, wherein said composition comprises polyisobutene as sole arthropodicidally-active ingredient.

8. Composition according to claim 7, wherein said polyisobutene is comprised in an amount of 0.1 to 25.0 % by weight of the composition, preferably in an amount of 0.5 to 10.0% by weight of the composition, most preferably in an amount of 2.0 to 6.0% by weight of the composition.

9. Composition according to any of claims 7-8, wherein said hydrophobic carrier is comprised of C6-C24 hydrocarbons, preferably C10-C20 hydrocarbons, most preferably C14-C19 isoparaffinic oil.

10. Composition according to any of claims 7-9, wherein said composition further comprises a film-forming polymer, preferably said film-forming polymer is an acrylate polymer or acrylate copolymer.

11. Composition according to any of claims 7-10, wherein said composition is a lotion.

12. Composition according to claim 11, wherein said acrylate polymer or said acrylate copolymer is comprised in an amount of 0.1 to 2.0 % by weight of the composition.

13. Composition according to any of claims 7-11, wherein said composition comprises sesame oil.

14. Composition according to any of claims 7-13, for use in treatment or prevention of infection of arthropod eggs.

15. Method for preparing a composition according to any of claims 7-14, comprising the step of mixing at least one polyisobutene according to any of claims 1-6 with a hydrophobic carrier.
